(19) Europäisches Patentamt
European Patent Office
Office européen des brevets

(11) Publication number: **0 028 641**
**B1**

(12) **EUROPEAN PATENT SPECIFICATION**

(45) Date of publication of patent specification: **24.04.85**

(51) Int. Cl.⁴: **A 61 K 31/72**

(21) Application number: **80901128.1**

(22) Date of filing: **08.05.80**

(86) International application number:
**PCT/US80/00527**

(87) International publication number:
**WO 80/02374 13.11.80 Gazette 80/26**

(54) **HYDROXYALKYL STARCH DRUG CARRIER.**

(30) Priority: **10.05.79 US 37981**

(43) Date of publication of application:
**20.05.81 Bulletin 81/20**

(45) Publication of the grant of the patent:
**24.04.85 Bulletin 85/17**

(84) Designated Contracting States:
**AT CH DE FR GB LI LU NL SE**

(56) References cited:
**DE-A-2 829 037**
**DE-A-2 838 736**
**DE-B-1 167 794**
**GB-A-1 495 159**
**US-A-3 908 004**
**US-A-3 928 581**
**US-A-4 026 986**
**US-A-4 180 567**

**Science, vol. 157, 15 Sep 1967, C.T.KNORPP et al.: "Hydroxyethyl starch: Extracellular cryophlactic agent for erythrocytes", pp. 1312, 1313**

(73) Proprietor: **AMERICAN HOSPITAL SUPPLY CORPORATION**
**One American Plaza**
**Evanston, IL 60201 (US)**

(72) Inventor: **BERGER, Kenneth L.**
**1224 Brangwyn Way**
**Laguna Beach, CA 92651 (US)**
Inventor: **BRAKE, Jon M.**
**720 W. Foothill, Apt. 8**
**Monrovia, CA 91016 (US)**
Inventor: **DEINDOERFER, Fred H.**
**9221 Encino Avenue**
**Northridge, CA 91324 (US)**
Inventor: **BANKERT, Charles S.**
**5408 B Seashore Drive**
**Newport Beach, California 92663 (US)**

(74) Representative: **Seaborn, George Stephen et al**
***c/o Edward Evans & Co. Chancery House 53-64***
**Chancery Lane**
**London WC2A 1SD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 028 641 B1**

(56) References cited:
CHEMICAL ABSTRACTS, vol. 87, no. 16, 17th October, 1977, page 333, abstract 122803f, COLUMBUS, OHIO (US)

CHEMICAL ABSTRACTS, vol. 92, no. 4, 28th January, 1980, page 412, abstract 28562h, COLUMBUS, OHIO (US)

**Description**

Background of the invention

This invention relates to compositions of matter for the controlled release of a biologically active compound to a human or animal. More particularly, the invention relates to a polymeric carrier for such biologically active substances which has a low order of toxicity and long term persistence in the body.

Various methods for administering drugs in controlled release formulations, e.g. sustained release or delayed release, have been proposed. A concept which has been of general interest in the field involves administration of drugs as derivatives of a polymeric compound. The drug-polymer bond may be labile to be broken by chemical or biological action *in vivo* thus releasing the drug, or the drug-polymer bond may be substantially stable in the biological system. In the latter types of drug-polymer combinations, the drug remains active on the polymer (or polymer fragments). An advantage of administering drugs as drug-polymer combinations is that the activity of the drug can be controlled over a prolonged period of time, which is often not possible if the drug is administered in a single dose. This prolongation of activity of the drug is dependent on, among other factors, the strength of the polymer-drug bond in the biological system and the rate of metabolism of the polymeric drug carrier. Another advantage of administering drugs in such a manner is that the toxicity of the drug is often reduced. Certain drugs, notably chemotherapeutic agents used for treatment of certain neoplastic diseases, are quite toxic, and methods to reduce their toxicity while maintaining their activity are very desirable. This reduction in toxicity can be a result of a diminution of the effective concentration of the drug in the biological fluid since its release or activity in that fluid occurs over a longer period of timne. Frequently, such drugs are metabolized or excreted very rapidly; therefore, to obtain a desired level of activity, the physician must administer relatively large doses. By utilizing a drug-polymer combination, the rate of metabolism or excretion of the drug may be reduced, thus lowering the actual dose requirements. Another reason that the toxicity may be lower is that a drug which might otherwise undergo undesirable reactions in the body, such as precipitation, complex formation, reaction or degradation, will be prevented or inhibited from such reactions as a result of the polymer-drug combination.

Specific polymers which have been employed as drug carriers are dextran, which is a polysaccharide obtained by the fermentation of sucrose, and various synthetic polymers such as vinyl polymers, polyacrylates and polyamides. Examples of drug-polymer complexes employing dextran are disclosed by Herb, J. R., U.S. Patent 2,885,393, May 5, 1959 and London, E., et al., U.S. Patent Re. 24,642, April 28, 1959. Polymer drug complexes are described generally in an article published in *Chemical Week*, April 26, 1978, page 45. The following United States patents also describe combinations between polymers and various active components:

3,608,063
3,629,392
3,966,902
3,998,620
3,998,974
4,003,990
4.035,479

To be useful as a drug carrier, a polymer must be capable of forming a bond or complex with the drug. This capability is dependent on the reactive bonding sites on the drug, and also on the bonding ability of the polymer. In the case of a polymer-drug combination in which the drug is released from the polymer *in vivo*, the drug and polymer should be connected by a labile bond. In the case of a combination in which the drug remains on the polymer *in vivo*, the drug-polymer bond must be relatively stable and must not appreciably interfere with the drug activity. The polymer must itself also be substantially non-toxic. Advantageously, the polymer can be modified so that the rate of release of the drug component or its *in vivo* activity can be accurately controlled. A disadvantage of several of the polymers which have been investigated for this purpose is that they persist in the body. That is, after the drug groups have been released, the polymers are not easily excreted or metabolized to harmless components. Such polymers may, therefore, act as cumulative poisons in the body and defeat their desired function.

Generally, with some exceptions, the use of polymeric drug carriers has heretofore been limited to formulations for oral or topical administration. The toxic effects and the degree of long term persistence of the polymer in the body are much more significant in parenteral administration. The polymers for oral or topical use are generally unsatisfactory for parenteral use because of these factors. Although dextran has been proposed as a drug carrier for parenteral administration, its use has caused undesirable reactions. In particular, dextran has been found to be antigenic and its use has caused anaphylaxis in patients.

Accordingly, there is a need for a drug carrier polymer which can be easily modified to control the rate of drug release, which inherently has a low order of toxicity, and which, subsequent to drug release, is quickly and substantially excreted from the body or metabolized into harmless components.

Summary of the invention

In accordance with the present invention, there is provided a composition of matter for the controlled

release of a biologically active compound to a human or animal, comprising the biologically active compound chemically bonded to hydroxyalkyl starch capable of being metabolized by or excreted from the human or animal body.

Detailed description of the invention

The preferred polymeric drug carrier of this invention is a hydroxyalkyl starch which can be prepared in accordance with the teachings of Hershenson, H., et al., U.S. Patent 3,523,938, August 11, 1970, incorporated herein by reference. The preferred polymeric material is hydroxyethyl starch which is prepared by etherifying waxy starch with ethylene oxide to a predetermined substitution level, and then hydrolyzing the etherified starch to a desired viscosity range. Substitution level or degree is defined as the number of moles of substituent groups (i.e. hydroxyalkyl groups) per mole of anhydroglucose units in the starch.

The Hershenson patent discloses the use of hydroxyalkyl starch as a plasma expander. One of the properties of the polymer which makes it particularly suited for that purpose is that the compound has a high short term persistence in the body, but has a very low long term persistence. Thus, after the polymer has served its function, it is substantially metabolized or excreted from the body, resulting in little, if any, toxic build up.

This property of low long term *in vivo* persistence also enables hydroxyalkyl starch to be advantageously used as a drug carrier. After the drug or biologically active compound has been released in the body, or the effect thereof has been realized, the polymer is substantially metabolized or excreted. Side effects resulting from the use of the drug carrier are thereby minimized.

The period of prolongation of the activity of the active component is somewhat dependent on the degree of persistence of the polymer. This degree of persistence can, in turn, be controlled by varying the level of substitution of the polymer as described by Hershenson, et al., supra. The greater the degree of substitution, the slower will be the rate of starch hydrolysis *in vivo*. If the active component is effective while bound to the polymer or to polymer subunits, higher degrees of substitution will prolong such effectiveness by increasing the degree of persistence of the polymer. If shorter action is desired, the degree of substitution can be reduced, resulting in more rapid hydrolysis of the polymer and thus more rapid release of the active component. If the active compound is bound to the polymer by a labile bond, the rate of release of such compound into a body fluid is dependent both upon the level of substitution of the polymeric carrier and upon the strength of the labile bond *in vivo*.

The preferred level of substitution of the hydroxyalkyl starch will vary, depending upon the particular active component for which the polymer is a carrier, the rate of release or prolongation of activity which is desired, upon the manner of administration. The level will generally fall in the range of from 0.1 to 3 for oral administration and from 0.1 to 1 for parenteral administration. The preferred range of the level of substitution is from 1 to 3 for oral administration and from 0.4 to 0.8 for parenteral administration.

It should be recognized that the hydrolysis of hydroxyalkyl starch is accomplished in the body largely by enzyme action, e.g. by the action of amylase. Thus, the degree of persistence of the polymer is increased as substituent groups thereon are added. As indicated, the level of substitution with hydroxyethyl groups may advantageously be used to control the degree of persistence, but substitution with the biologically active component can also affect the susceptability of the polymer to enzyme action, and thus determine the degree of persistence. In an extreme case, the level of substitution of the polymer may be very low, approaching zero, but because of the presence of active compound on the polymer, the degree of persistence is in the desired range. Because of the effect of the active compound substituent on the level of persistence, the level of substitution of the polymer and the number of active compound substituents thereon should be determined, based on the degree of persistence desired in each individual case.

The molecular weight of the hydroxyalkyl starch also affects its persistence in the body and the availability of the active component. The molecular weight can be controlled by regulating the degree of acid hydrolysis as taught by Hershenson, et al., supra. For parenteral administration, the molecular weight of the polymer is advantageously between 1,000 and 500,000, preferably between 10,000 and 200,000. Higher molecular weights are usually employed for oral or topical administration and generally range from 10,000 to 2,000,000 and are preferably in a range of from 200,000 to 450,000.

The chemical bonding of the biologically active compound to the hydroxyalkyl starch may be subject to cleavage *in vivo* or may be substantially stable *in vivo*, the biologically active compound maintaining biological activity *in vivo* while bonded to the hydroxyalkyl starch. By "substantially stable *in vivo*" in relation to the chemical bonding of the biologically active compound to the hydroxyalkyl starch is meant that the rate of cleavage of the chemical bonding *in vivo* is low compared with the rate of hydrolysis of the starch *in vivo*.

In accordance with the present invention, it has been discovered that a wide variety of biologically active components can be combined with hydroxyalkyl starch to be released *in vivo* in a controlled manner, e.g. sustained release or delayed release. As used herein, the term controlled release shall include the actual release of the active compound into the body over a period of time, and shall also include a prolongation or modification of the *in vivo* activity of such compound although it remains bound to the polymeric carrier or fraction thereof. Such biologically active components can be combined with the

polymer directly or through suitable derivatives by chemical bonds, e.g. covalent or ionic bonds, complexation or other means.

Hydroxyalkyl starch, being a substituted polysaccharide, has a plurality of hydroxyl groups which provide useful sites for bonding active compounds. Such bonding is not limited, however, to reactions with the hydroxyl groups. This bonding can be a direct reaction between the active component and the polymer. For instance, if the active component has a carboxylic acid functional group, it can react directly or indirectly with a hydroxyl group on the hydroxyalkyl starch to form an ester. The ester linkage can be easily cleaved *in vivo* by hydrolysis or enzyme action to release the active compound. In addition to being reacted directly with the polymer, the active compound can be bound to the polymer through a derivative. For example, the following schemes may be employed, where R is an appropriately selected derivatizing agent:

Scheme I

$$\text{Polymer} + R \rightarrow \text{Polymer} - R$$
$$\text{Drug} + R^1 \rightarrow \text{Drug} - R^1$$
$$\text{Polymer} - R + \text{Drug} - R^1 \rightarrow \text{Polymer} - R - R^1 - \text{Drug}$$

Scheme II

$$\text{Polymer} + R \rightarrow \text{Polymer} - R$$
$$\text{Polymer} - R + \text{Drug} \rightarrow \text{Polymer} - R - \text{Drug}$$

Scheme III

$$\text{Drug} + R \rightarrow \text{Drug} - R$$
$$\text{Drug} - R + \text{Polymer} \rightarrow \text{Drug} - R - \text{Polymer}$$

In each of the above schemes, the derivatizing agents are carefully selected so that the drug or an active drug derivative will be released *in vivo*, or the activity of the drug will be maintained while it is bound to the polymer. A fourth scheme involves the reaction of a drug precursor ($\text{Drug}_p$) with a derivative, followed by reaction with the hydroxyalkyl starch polymer. Upon reaction *in vivo*, the active drug is released. This scheme is represented below.

$$\text{Drug}_p + R \rightarrow \text{Drug}_p - R$$
$$\text{Drug}_p - R + \text{Polymer} \rightarrow \text{Drug}_p - R - \text{Polymer}$$
$$\text{Drug}_p - R - \text{Polymer} \xrightarrow{\text{in vivo}} \text{Drug}$$

Derivatizing agents useful for producing compositions of this invention include substantially any non-toxic compound which will link the active compound to the polymer. Polyfunctional organic compounds are useful for this purpose. Through the above-described reaction schemes, a wide variety of biologically active compounds can be combined with hydroxyalkyl starch to form controlled release formulations.

Specific examples of useful modifications to hydroxyalkyl starch are listed below (HES indicates hydroxyethyl starch). For convenience, the reaction involving only one or two hexose units of the hydroxyethyl starch is indicated.

(1)

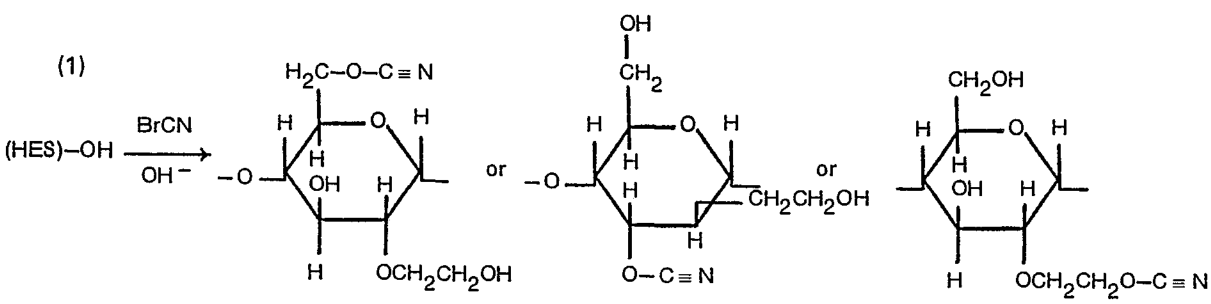


These hydroxyethyl starch derivatives can be reacted with proteins such as the antiviral agent interferon, peptides such as the enkephalins, and amino acids.

(2) Drugs having reactive hydroxyl groups may be derivatized to react with hydroxyethyl starch through stable ether groups in accordance with the following scheme (HO—R represents the drug):

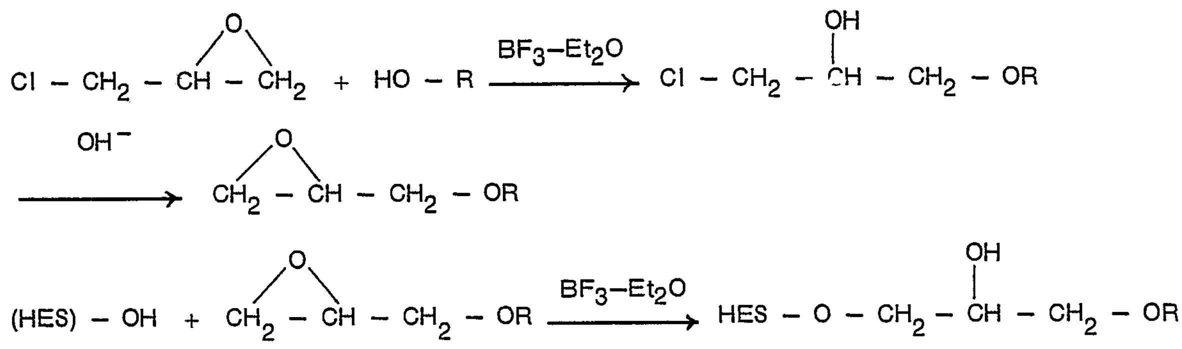


Examples of drugs having such hydroxyl groups include steroids such as hydrocortisone and prednisone and antibiotics such as chloramphenicol.

(3) Hydroxyethyl starch can be halogenated to react in a wide variety of ways. The halogenated derivative may be formed during the preparation of the polymer or by direct halogenation. The following reactions may be employed:

A. 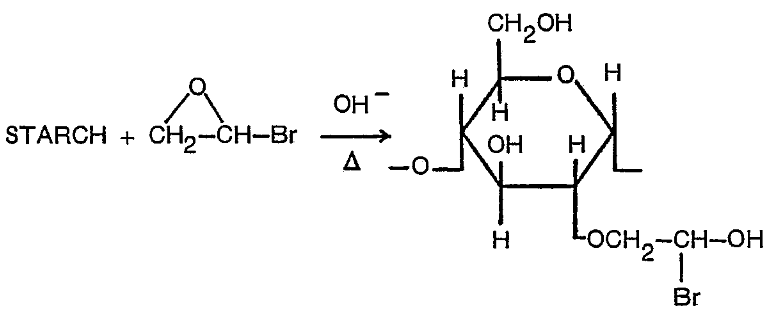


and 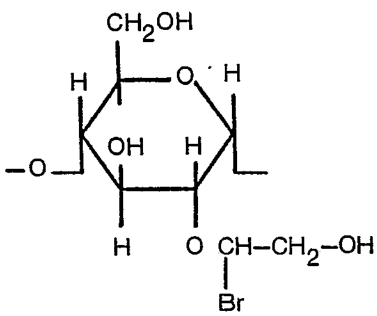

B. 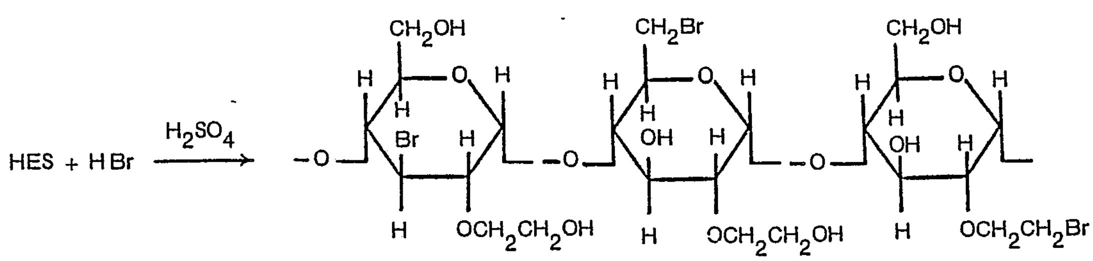


(4) Drugs having alkyl halide functions, such as the antineoplastic agents chlorambucil and cyclophosphamide

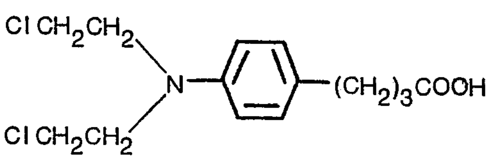

chlorambucil

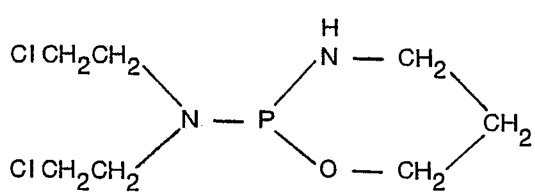

cyclophosphamide

can react with hydroxyethyl starch directly by the following reaction:

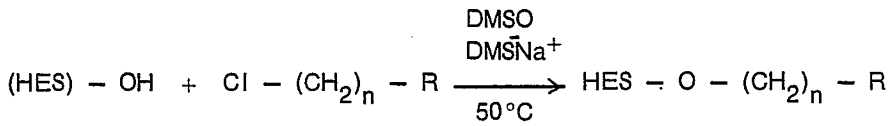


(5) Brominated hydroxyethyl starch can be used as a precursor for a Grignard reagent which in turn can be reacted with drugs having aldehyde or ketone functional groups.

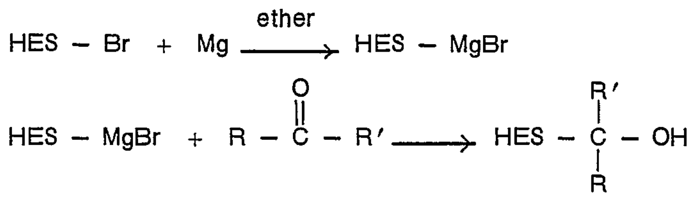


The endocrine antagonist, aldosterone, is an example of a ketone which can participate in the above reaction.

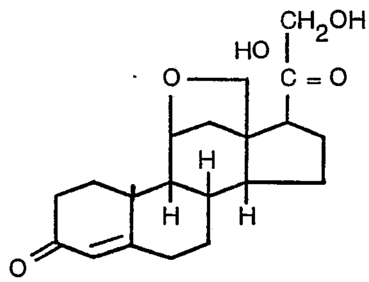

aldosterone

(6) Drugs such as chloramphenicol containing the

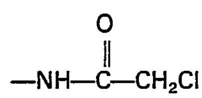

groups can be reacted directly with hydroxyethyl starch by the following reaction:

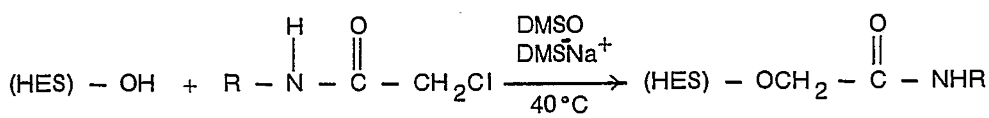


The reaction is therefore useful for any drug having an acetamide group which can be chlorinated, e.g. sulfacetamide and the antimalarial agent DADDS (4,4'-diacetyl-4,4'-diaminodiphenylsulfone).

(7) Active compounds having carboxylic acid functional groups can be converted to acyl halides by reaction with thionyl chloride. For instance, chlorambucil can react as follows:

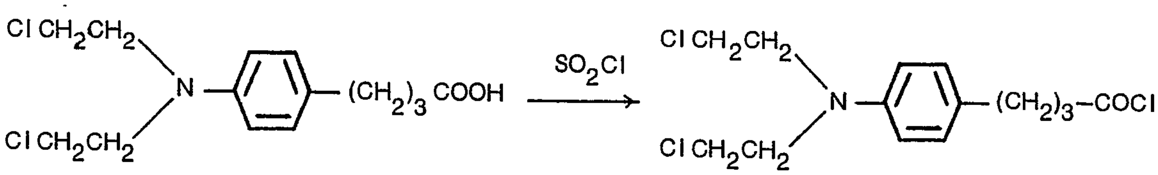


Acyl halides can, in turn, react with hydroxyethyl starch as follows ($E + Cl_2$ represents 1,2-dichloroethane):

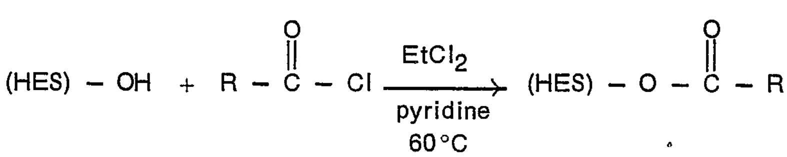

(8) Drugs having alkyl halide groups such as the antineoplastic agent pipobroman can react to form isocyanates which can react with hydroxyethyl starch.

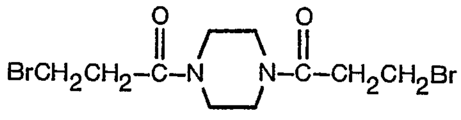

pipobroman

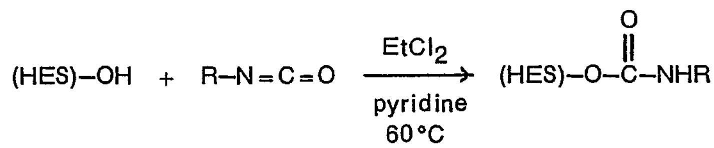


(9) Anhydrides can react with hydroxyethyl starch in the following manner:

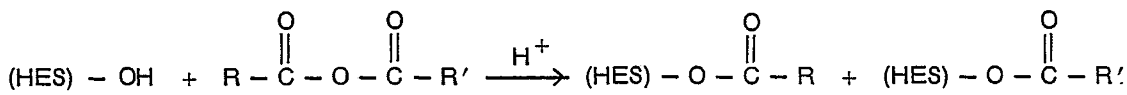


Thus, drugs having carboxylic acid groups, such as vitamin $A_2$ can be reacted with acetic anhydride to form a mixed anhydride which, in turn, can be so reacted with hydroxyethyl starch.

(10) A wide variety of drugs have amine functional groups. Included in this group are amphetamines and dopamine.

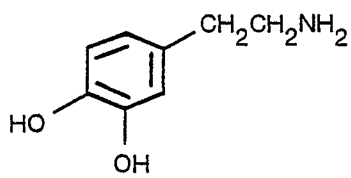

Dopamine

Hydroxyethyl starch can be partially oxidized to form aldehydic compounds which can react with such amine groups to form Schiff base compounds.

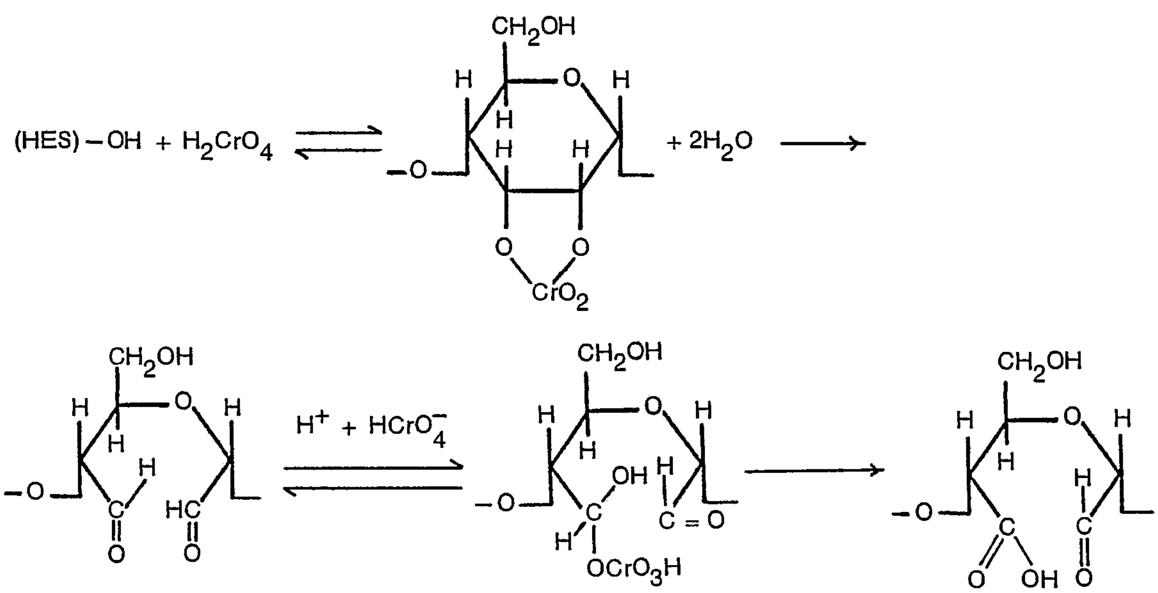


another useful oxidation reaction is:

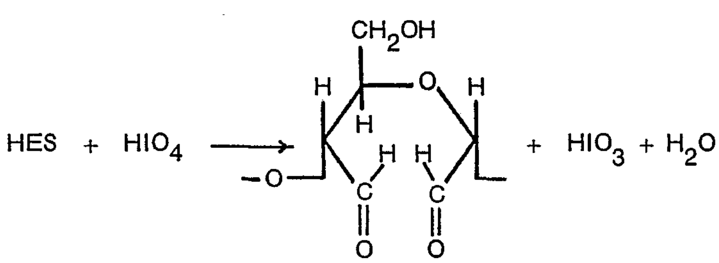

The reaction of aldehydic hydroxyethyl starch with an amine-containing drug is represented as follows:

$$(HES) - \overset{\overset{\displaystyle O}{\|}}{C} - H + H_2N - DRUG \longrightarrow HES - CH = N - Drug$$

Certain drugs can be derivatized to contain amine groups which can react with aldehydic hydroxyethyl starch by this scheme.

The present invention is particularly advantageous for administering iron to a patient. The requirements of the body for iron and its therapeutic and prophylactic uses are well documented. Iron salts are generally not administered orally for therapeutic purposes because they are poorly absorbed or because they sometimes cause disturbances in the alimentary tract. Therefore, iron is preferably administered parenterally, generally by intravenous injection. Solutions of iron salts are not usually injected directly because they are toxic. A particular problem is that acidic iron forms insoluble precipitates at physiological pH. To overcome these problems, physiologically compatible iron complexes have been developed for parenteral administration. Such complexes have included saccharated iron, complexes of iron with dextrans or dextrins, and complexes of iron with a water swellable polymer. Examples of the latter complexes are described in U.S. Patent 2,885,393, May 5, 1959, and Canadian Patent 991,544.

It has been found that the present invention may be advantageously employed for the administration of iron. Citric acid has a stabilizing effect on the iron and prevents precipitate formation during the reaction. The iron may be bound to the hydroxyalkyl starch through a citric acid derivative, but the exact structure of the iron-polymer combination is not presently known. The iron is provided by any soluble salt, preferably in the ferric form. An iron salt, such as ferric chloride, and citric acid is combined with an aqueous solution of hydroxyalkyl starch. The solution is advantageously clarified and the pH is raised, e.g. by addition of aqueous ammonia. The resulting solution of iron-hydroxyalkyl starch complex is purified (de-salted) by any convenient means, such as dialysis or ion exchange. The iron-hydroxyalkyl starch complex may be stored as a sterile solution, or may be precipitated and stored in dry form.

The iron-hydroxyalkyl starch compounds of this invention provide iron in safe effective concentrations, and after release of the iron *in vivo*, the remaining hydroxyalkyl starch is excreted or metabolized. Thus, the compounds can be continuously or repeatedly administered over prolonged periods with few or no side effects attributable to the polymer carrier.

The combinations produced in accordance with the present invention can be administered either orally, parenterally or topically. The low order of *in vivo* persistence of the hydroxyalkyl starch carrier is, of course, most appreciated during parenteral and particularly during intravenous administration. To the drug-polymer combination may be added conventional pharmaceutical excipients. For instance, intravenous solutions generally contain electrolytes and pH controlling agents to insure that physiological conditions of osmotic pressure and pH exist. Such solutions may also contain nutrients, such as glucose and amino acids as well as other active compounds. Oral formulations may contain conventional excipients, such as flavoring agents and compounds useful for suspending the drug-polymer combination in a liquid or compounds for forming the combination into tablets or capsules. Methods for formulating drugs are well known in the pharmaceutical arts, and the present invention is not limited to particular formulations.

Thus, described herein are novel combinations of hydroxyalkyl starch and biologically active compounds and a method for their administration. Such combinations are characterized by a low long term persistence of the hydroxyalkyl starch in the body and by controlled release of the active compound *in vivo*.

The invention is further illustrated by the following examples, but is not intended to be limited thereby.

## Example I

This Example demonstrates the preparation of an iron-hydroxyethyl starch combination.

Hydroxyethyl starch (prepared by the procedure described in Example II of U.S. Patent 3,523,938, and further acid hydrolyzed to a molecular weight of 45,000), 80 g, was dissolved in water to yield 400 ml. of a 20% w/v solution (Solution I). Ferric chloride ($FeCl_3 \cdot 6H_2O$), 125 g, was dissolved in water to yield 250 ml. of a 50% w/v solution (Solution II). Solution I was heated to 60°C with stirring, and Solution II was heated to 40—60°C with stirring. Solution II was slowly added to Solution I with stirring. After addition was complete, granular citric acid, 53.5 g, was slowly added to the mixture with stirring, and the resulting solution was stirred at 60°C for an additional 20 minutes then allowed to cool to room temperature. A 20% aqueous solution of $NH_4OH$ was prepared (Solution III) and slowly added with stirring to the iron-hydroxyethyl starch-citric acid solution until the pH reached 10.4. The solution was then heated to 50°C and stirred for 20 minutes, cooled to room temperature and filtered through a 0.8 μ filter. The solution was purified by overnight dialysis against distilled water, and was concentrated to 1100 ml. by vacuum distillation. The iron-polymer combination was precipitated by combining the concentrated solution with 8 liters of acetone. The resulting precipitate was collected by filtration and washed several times with acetone. The precipitate was dried at 80°C *in vacuo*, yielding 91 grams of dry product having an iron content of 22.7 by weight.

Example II

The product from Example I was used for the preparation of an injection solution. The dry product (50 g) was dissolved in warm (50°C) sterile water (100 ml.) by adding the powder slowly to the warm water with stirring. The solution was brought to 175 ml. by the addition of 1.58 g of NaCl and sterile water. The solution was then filtered through a 0.22 μ filter and placed in 30 ml. vials. The vials were sterilized for 15 minutes and 250°F (121°C) and the solution has an iron concentration of 50.5 mg/ml.

Example III

The experiment of Example I was repeated in all essential details except that following dialysis and concentration of the solution, the iron-hydroxyethyl starch was precipitated in cold (5—10°C) acetone, the supernatant discarded and the precipitate washed with one liter of 80% aqueous acetone. The precipitate was allowed to resettle, the supernatant discarded, and the procedure repeated. After the second wash, the precipitate was redissolved in 400 ml. of a 0.7% w/v solution of citric acid. The pH was then adjusted to 8.1 with 4N $NH_4OH$, and the solution was diluted to 600 ml. with water. The iron-hydroxyethyl starch was reprecipitated and washed with two 1 liter aliquots of 80% acetone. The resulting precipitate was then redissolved as above, reprecipitated, washed with three 1 liter aliquots of 100% acetone and collected by vacuum filtration. The collected precipitate was washed with an additional 1 liter of 100% acetone during filtration and was dried as described in Example I.

Example IV

A dry preparation of iron-hydroxyethyl starch was prepared as follows: Hydroxyethyl starch, 10 g, was dissolved in 100 ml. of water. Ferric chloride ($FeCl_3 \cdot 6H_2O$), 20 g, was added to the hydroxyethyl starch solution with stirring, and the resulting solution was heated to 50°C for 15 minutes then heated to 70°C for 30 minutes. The solution was allowed to cool to room temperature and the pH was adjusted from 1.4 to 2.9 by addition of 20% $NH_4OH$. The resulting solution was then dialyzed against running distilled water overnight. The dialyzed solution was concentrated by rotary evaporation to 80 ml., and the concentrated solution was combined with 1200 ml. of acetone which had been cooled to −20°C by the addition of dry ice, thus causing precipitation of the iron-hydroxyethyl starch. The resulting slurry was allowed to warm to room temperature with stirring, and the precipitate was collected by vacuum filtration; the precipitate being washed with 1 liter of acetone in the process. The precipitate was then dried in a vacuum oven at 90°C overnight to yield 9.8 g of dry product.

Example V

The experiment of Example I was repeated in all essential details except that the hydroxyethyl starch starting material was subjected to acid hydrolysis to a molecular weight of 10,000. The solution was purified by ultrafiltration through a 1000 molecular weight cut off filter to remove free ions. The dialysis step was excluded from the procedure. The experiment yielded 80 g of dry product containing 25.4% iron by weight.

Example VI

This example describes the preparation of a hydroxyethyl starch-insulin combination in accordance with the present invention. Hydroxyethyl starch (1 g) in 25 ml. of water is added to a well stirred mixture of CNBr (200 mg) in 100 ml. of water. The pH is maintained at 11 by the addition of 2N NaOH. The activation reaction is continued for 10 minutes and 20 mg of insulin in 20 ml. of 1 M sodium bicarbonate are then rapidly added, lowering the pH. The solution is then stirred overnight in an ultrafiltration cell equipped with an appropriate membrane. The solution is then concentrated and washed with 6 Molal guanidine hydrochloride. When no further fee insulin is detected migrating through the membrane, the composition is thoroughly washed with water and concentrated to a final volume of 60—80 ml. The experiment should yield a hydroxyethyl starch-insulin combination useful for the controlled release administration of insulin.

Example VII

The experiment of Example VI is repeated in all essential details, except a mixture of amino acids is substituted for insulin. The experiment should yield a hydroxyethyl starch-amino acids combination useful for the controlled release administration of amino acids.

Example VIII

The experiment of Example VII is repeated in all essential details except enkephalin (peptide) is substituted for insulin. The experiment should yield a hydroxyethyl starch-enkephalin combination useful for the controlled release administration of enkephalin.

Example IX

The experiment of Example I is repeated in all essential details except hydroxypropyl starch is substituted for hydroxyethyl starch. The experiment should yield an iron-hydroxypropyl starch combination.

**Claims for the Contracting States: CH DE FR GB LI LU NL SE**

1. A composition of matter for the controlled release administration of a biologically active compound to a human or animal, comprising the biologically active compound chemically bonded to hydroxyalkyl starch capable of being metabolized by or excreted from the human or animal body.

2. The composition of claim 1, wherein the chemical bonding is subject to cleavage *in vivo*.

3. The composition of claim 1, wherein the chemical bonding is substantially stable *in vivo*, and said biologically active compound maintains biological activity *in vivo* while bonded to said hydroxyalkyl starch.

4. The composition of claim 2 or 3, wherein the biologically active compound is a drug.

5. The composition of claim 2 or 3, wherein the hydroxyalkyl starch is hydroxyethyl starch.

6. The composition of claim 2 or 3, wherein the biologically active compound is bound to the hydroxyalkyl starch through a derivative.

7. The composition of claim 5, wherein said composition is for oral administration, and said hydroxyethyl starch has a level of substitution of from 0.1 to 3.

8. The composition of claim 5, wherein said composition is for oral administration, and said hydroxyethyl starch has a level of substitution of from 1 to 3.

9. The composition of claim 5, wherein said composition is for parenteral administration, and said hydroxyethyl starch has a level of substitution of from 0.1 to 1.

10. The composition of claim 5, wherein said composition is for parenteral administration, and said hydroxyethyl starch has a level of substitution of from 0.4 to 0.8.

11. The composition of claim 7 or 8, wherein the molecular weight of said hydroxyethyl starch is from 10,000 to 2,000,000.

12. The composition of claim 7 to 8, wherein the molecular weight of said hydroxyethyl starch is from 200,000 to 450,000.

13. The composition of claim 9 or 10, wherein the molecular weight of said hydroxyethyl starch is from 1,000 to 500,000.

14. The composition of claim 9 or 10, wherein the molecular weight of said hydroxyethyl starch is from 10,000 to 200,000.

15. The composition of claim 1 or 2, wherein the biologically active compound comprises iron.

16. The composition of claim 15, further comprising citric acid.

17. A composition of matter according to any preceding claim, for use in a method of treatment of the human or animal body by therapy.

18. A composition of matter according to any preceding claim, wherein the biologically active compound is insulin, hydrocortisone, prednisone, interferon, an antibiotic, an enkephalin or other peptide, dopamine or an antineoplastic agent.

**Claims for the Contracting State: AT**

1. A method of preparing a composition of matter for the controlled release administration of a biologically active compound to a human or animal, comprising chemically bonding the biologically active compound directly or indirectly to hydroxyalkyl starch capable of being metabolized by or excreted from the human or animal body.

2. The method of claim 1, wherein the chemical bond formed between the biologically active compound and the hydroxyalkyl starch is such as to be subject to cleavage *in vivo*.

3. The method of claim 1, wherein the chemical bond formed between the biologically active compound and the hydroxyalkyl starch is such as to be stable *in vivo*, the biologically active compound maintaining biological activity *in vivo* while bonded to the hydroxyalkyl starch.

4. The method of claim 2 or 3, wherein the biologically active compound is a drug.

5. The method of claim 2 or 3, wherein the hydroxyalkyl starch is hydroxyethyl starch.

6. The method of claim 2 or 3, wherein the biologically active compound is bound to the hydroxyalkyl starch through a derivative.

7. The method of claim 5, wherein said composition is for oral administration, and said hydroxyethyl starch has a level of substitution of from 0.1 to 3.

8. The method of claim 5, wherein said composition is for oral administration, and said hydroxyethyl starch has a level of substitution of from 1 to 3.

9. The method of claim 5, wherein said composition is for parenteral administration, and said hydroxyethyl starch has a level of substitution of from 0.1 to 1.

10. The method of claim 5, wherein said composition is for parenteral administration, and said hydroxyethyl starch has a level of substitution of from 0.4 to 0.8.

11. The method of claim 7 or 8, wherein the molecular weight of said hydroxyethyl starch is from 10,000 to 2,000,000.

12. The method of claim 7 or 8, wherein the molecular weight of said hydroxyethyl starch is from 200,000 to 450,000.

13. The method of claim 9 or 10, wherein the molecular weight of said hydroxyethyl starch is from 1,000 to 500,000.

14. The method of claim 9 or 10, wherein the molecular weight of said hydroxyethyl starch is from 10,000 to 200,000.

15. The method of claim 1 or 2, wherein the biologically active compound comprises iron.

16. The method of claim 15, further comprising incorporating citric acid into the composition.

17. The method of any preceding claim, wherein the biologically active compound is insulin, hydrocortisone, prednisone, interferon, an antibiotic, an enkephalin or other peptide, dopamine or an antineoplastic agent.

**Patentansprüche für die Vertragsstaaten: CH DE FR GB LI LU NL SE**

1. Präparat aus einem Stoff für die Verabreichung einer biologisch aktiven zusammengesetzten Substanz an einen Menschen oder ein Tier mit kontrollierter Freisetzung, die umfaßt, daß die biologisch aktive zusammengesetzte Substanz chemisch an Hydroxyalkylstärke gebunden ist, die durch den menschlichen oder tierischen Körper umgewandelt oder aus diesem ausgeschieden werden kann.

2. Präparat nach Anspruch 1, bei dem die chemische Bindung einer Spaltung in vivo unterliegt.

3. Präparat nach Anspruch 1, bei dem die chemische Bindung in vivo im wesentlichen stabil ist und die biologisch aktive zusammengesetzte Substanz die biologische Aktivität in vivo beibehält, während sie an die Hydroxyalkylstärke gebunden ist.

4. Präparat nach Anspruch 2 oder 3, bei dem die biologisch aktive zusammengesetzte Substanz ein Arzneimittel ist.

5. Präparat nach Anspruch 2 oder 3, bei dem die Hydroxyalkylstärke Hydroxyäthylstärke ist.

6. Präparat nach Anspruch 2 oder 3, bei dem die biologisch aktive zusammengesetzte Substanz durch ein Derivat an die Hydroxyalkylstärke gebunden ist.

7. Präparat nach Anspruch 5, bei dem das Präparat zur oralen Verabreichung vorgesehen ist und die Hydroxyäthylstärke einen Substitutionsgrad von 0.1 bis 3 hat.

8. Präparat nach Anspruch 5, bei dem das Präparat zur oralen Verabreichung vorgesehen ist und die Hydroxyäthylstärke einen Substitutionsgrad von 1 bis 3 hat.

9. Präparat nach Anspruch 5, bei dem das Präparat zur parenteralen Verabreichung vorgesehen ist und die Hydroxyäthylstärke einen Substitutionsgrad von 0.1 bis 1 hat.

10. Präparat nach Anspruch 5, bei dem das Präparat zur parenteralen Verabreichung vorgesehen ist und die Hydroxyäthylstärke einen Substitutionsgrad von 0.4 bis 0.8 hat.

11. Präparat nach Anspruch 7 oder 8, bei dem das Molekulargewicht der Hydroxyäthylstärke von 10000 bis 2000000 beträgt.

12. Präparat nach Anspruch 7 oder 8, bei dem das Molekulargewicht der Hydroxyäthylstärke von 2000000 bis 450000 beträgt.

13. Präparat nach Anspruch 9 oder 10, bei dem das Molekulargewicht der Hydroxyäthylstärke von 1000 bis 500000 betragt.

14. Präparat nach Anspruch 9 oder 10, bei dem das Molekulargewicht der Hydroxyäthylstärke von 10000 bis 2000000 beträgt.

15. Präparat nach Anspruch 1 oder 2, bei dem die biologisch aktive zusammengesetzte Substanz Eisen enthält.

16. Präparat nach Anspruch 15, das weiter Zitronensäure enthält.

17. Präparat aus einem Stoff nach einem beliebigen vorhergehenden Anspruch zur Verwendung in einem Verfahren zur therapeutischen Behandlung des menschlichen oder tierischen Körpers.

18. Präparat aus einem Stoff nach einem beliebigen vorhergehenden Anspruch, bei dem die biologisch aktive zusammengesetzte Substanz Insulin, Hydrocortision, Prednison, Interferon, ein Antibiotikum, ein Encephalin oder ein anderes Peptid, Dopamin oder ein antineoplastisches Mittel ist.

**Patentansprüche für den Vertragsstaat: AT**

1. Verfahren zur Herstellung eines Präparats aus einem Stoff für die Verabreichung einer biologisch aktiven zusammengesetzten Substanz an einen Menschen oder ein Tier mit kontrollierter Freisetzung, das umfaßt, daß die biologisch aktive zusammengesetzte Substanz direkt oder indirekt mit Hydroxyalkylstärke chemisch gebunden wird, die durch den menschlichen oder tierischen Körper umgewandelt oder aus diesem ausgeschieden wird.

2. Verfahren nach Anspruch 1, bei dem die zwischen der biologisch aktiven zusammengesetzten Substanz und der Hydroxyalkylstärke gebildete chemische Bindung derarat ist, daß sie einer Spaltung in vivo unterliegt.

3. Verfahren nach Anspruch 1, bei dem die zwischen der biologisch aktiven zusammengesetzten Substanz und der Hydroxyalkylstärke gebildete chemische Bindung derart ist, daß sie in vivo stabil ist, wobei die biologisch aktive zusammengesetzte Substanz die biologische Aktivität in vivo beibehält, während sie an die Hydroxyalkylstärke gebunden ist.

4. Verfahren nach Anspruch 2 oder 3, bei dem die biologisch aktive zusammengesetzte Substanz ein Arzneimittel ist.

5. Verfahren nach Anspruch 2 oder 3, bei dem die Hydroxyalkylstärke Hydroxyäthylstärke ist.

6. Verfahren nach Anspruch 2 oder 3, bei dem die biologisch aktive zusammengesetzte Substanz durch ein Derivat an die Hydroxyalkylstärke gebunden ist.

7. Verfahren nach Anspruch 5, bei dem die das Präparat zur oralen Verabreichung vorgesehen ist und die Hydroxyäthylstärke einen Substitutionsgrad von 0.1 bis 3 hat.

8. Verfahren nach Anspruch 5, bei dem das Präparat zur oralen Verabreichung vorgesehen ist und die Hydroxyäthylstärke einen Substitutionsgrad von 1 bis 3 hat.

9. Verfahren nach Anspruch 5, bei dem das Präparat zur parenteralen Verabreichung vorgesehen ist und die Hydroxyäthylstärke einen Substitutionsgrad von 0.1 bis 1 hat.

10. Verfahren nach Anspruch 5, bei dem das Präparat zur parenteralen Verabreichung vorgesehen ist und die Hydroxyäthylstärke einen Substitutionsgrad von 0.4 bis 0.8 hat.

11. Verfahren nach Anspruch 7 oder 8, bei dem das Molekulargewicht der Hydroxyäthylstärke von 10000 bis 2000000 beträgt.

12. Verfahren nach Anspruch 7 oder 8, bei dem das Molekulargewicht der Hydroxyäthylstärke von 2000000 bis 450000 beträgt.

13. Verfahren nach Anspruch 9 oder 10, bei dem das Molekulargewicht der Hydroxyäthylstärke von 1000 bis 500000 beträgt.

14. Verfahren nach Anspruch 9 oder 10, bei dem das Molekulargewicht der Hydroxyäthylstärke von 10000 bis 200000 beträgt.

15. Verfahren nach Anspruch 1 oder 2, bei dem die biologisch aktive zusammengesetzte Substanz Eisen enthält.

16. Verfahren nach Anspruch 15, das weiter umfaßt, daß Zitronensäure in das Präparat eingebaut wird.

17. Verfahren nach einem beliebigen vorhergehenden Anspruch, bei dem die biologisch aktive zusammengesetzte Substanz Insulin, Hydrocortison, Prednison, Interferon, ein Antibiotikum, ein Encephalin oder ein anderes Peptid, Dopamin, oder ein antineoplastisches Mittel ist.

**Revendications pour les Etats contractants: CH DE FR GB LI LU NL SE**

1. Composition de matière pour l'administration avec libération commandée d'un composé actif biologiquement à un être humain ou un animal, caractérisée en ce qu'elle comprend le composé actif biologiquement lié chimiquement à un amidon hydroxyalkylé capable d'être transformé par métabolisme par le ou excrété à partir du corps humain ou animal.

2. Composition suivant la revendication 1, caractérisée en ce que la liaison chimique est sujette à une séparation *in vivo*.

3. Composition suivant la revendication 1, caractérisée en ce que la liaison chimique est pratiquement stable *in vivo*, et ledit composé actif biologiquement maintient un activité biologique *in vivo* alors qu'il est lié audit amidon hydroxyalkylé.

4. Composition suivant la revendication 2 ou 3, caractérisée en ce que le composé actif biologiquement est un médicament.

5. Composition suivant la revendication 2 ou 3, caractérisée en ce que l'amidon hydroxyalkylé est de l'amidon hydroxyéthylé.

6. Composition suivant la revendication 2 ou 3, caractérisée en ce que le composé actif biologiquement est lié à l'amidon hydroxyalkylé par l'intermédiaire d'un dérivé.

7. Composition suivant la revendication 5, caractérisée en ce que ladite composition est destinée à une administration orale, et ledit amidon hydroxyéthylé possède un degré de substitution de 0,1 à 3.

8. Composition suivant la revendication 5, caractérisée en ce que ladite composition est destinée à une administration orale, et ledit amidon hydroxyéthylé possède un niveau de substitution de 1 à 3.

9. Composition suivant la revendication 5, caractérisée en ce que ladite composition est destinée à une administration parentérale, et ledit amidon hydroxyéthylé possède un niveau de substitution de 0,1 à 1.

10. Composition suivant la revendication 5, caractérisée en ce que ladite composition est destinée à une administration parentérale, et ledit amidon hydroxyéthylé possède un niveau de substitution de 0,4 à 0,8.

11. Composition suivant la revendication 7 ou 8, caractérisée en ce que le poids moléculaire dudit amidon hydroxyéthylé est de 10.000 à 2.000.000.

12. Composition suivant la revendication 7 ou 8, caractérisée en ce que le poids moléculaire dudit amidon hydroxyéthylé est de 200.000 à 450.000.

13. Composition suivant la revendication 9 ou 10, caractérisée en ce que le poids moléculaire dudit amidon hydroxyéthylé est de 1.000 à 500.000.

14. Composition suivant la revendication 9 ou 10, caractérisée en ce que le poids moléculaire dudit amidon hydroxyéthylé est de 10.000 à 200.000.

15. Composition suivant la revendication 1 ou 2, caractérisée en ce que ledit composé actif biologiquement comprend du fer.

16. Composition suivant la revendication 15, caractérisée en ce qu'elle comprend en outre de l'acide citrique.

17. Composition de matière suivant l'une quelconque des revendications précédentes, destinée à être utilisée dans un procédé de traitement du corps humain ou animal par thérapeutique.

18. Composition de matière suivant l'une quelconque des revendications précédentes, caractérisée en ce que le composé actif biologiquement est de l'insuline, de l'hydrocortisone, de la prednisone, de l'interféron, un antibiotique, une enképhaline ou autre peptide, de la dopamine ou un agent antinéoplastique.

**Revendications pour l'Etat contractant: AT**

1. Procédé de préparation d'une composition de matière pour l'administration avec libération commandée d'un composé actif biologiquement à un être humain ou un animal, caractérisé en ce qu'il comprend l'action de lier chimiquement le composé actif biologiquement directement ou indirectement à un amidon hydroxyalkylé capable d'être transformé par métabolisme par le ou excrété à partir du corps humain ou animal.

2. Procédé suivant la revendication 1, caractérisé en ce que la liaison chimique formée entre le composé actif biologiquement et l'amidon hydroxyalkylé est telle qu'elle soit sujette à une séparation *in vivo*.

3. Procédé suivant la revendication 1, caractérisé en ce que la liaison chimique formée entre le composé actif biologiquement et l'amidon hydroxyalkylé est telle qu'elle soit stable *in vivo*, le composé actif biologiquement maintenant une activité biologique *in vivo* alors qu'il est lié audit amidon hydroxyalkylé.

4. Procédé suivant la revendication 2 ou 3, caractérisé en ce que le composé actif biologiquement est un médicament.

5. Procédé suivant la revendication 2 ou 3, caractérisé en ce que l'amidon hydroxyalkylé est de l'amidon hydroxyéthylé.

6. Procédé suivant la revendication 2 ou 3, caractérisé en ce que le composé actif biologiquement est lié à l'amidon hydroxyalkylé par l'intermédiaire d'un dérivé.

7. Procédé suivant la revendication 5, caractérisé en ce que ladite composition est destinée à une administration orale, et ledit amidon hydroxyéthylé possède un degré de substitution de 0,1 à 3.

8. Procédé suivant la revendication 5, caractérisé en ce que ladite composition est destinée à une administration orale, et ledit amidon hydroxyéthylé possède un niveau de substitution de 1 à 3.

9. Procédé suivant la revendication 5, caractérisé en ce que ladite composition est destinée à une administration parentérale, et ledit amidon hydroxyéthylé possède un niveau de substitution de 0,1 à 1.

10. Procédé suivant la revendication 5, caractérisé en ce que ladite composition est destinée à une administration parentérale, et ledit amidon hydroxyéthylé possède un niveau de substitution de 0,4 à 0,8.

11. Procédé suivant la revendication 7 ou 8, caractérisé en ce que le poids moléculaire dudit amidon hydroxyéthylé est de 10.000 à 2.000.000.

12. Procédé suivant la revendication 7 ou 8, caractérisé en ce que le poids moléculaire dudit amidon hydroxyéthylé est de 200.000 à 450.000.

13. Procédé suivant la revendication 9 ou 10, caractérisé en ce que le poids moléculaire dudit amidon hydroxyéthylé est de 1.000 à 500.000.

14. Procédé suivant la revendication 9 ou 10, caractérisé en ce que le poids moléculaire dudit amidon hydroxyéthylé est de 10.000 à 200.000.

15. Procédé suivant la revendication 1 ou 2, caractérisé en ce que le composé actif biologiquement comprend du fer.

16. Procédé suivant la revendication 15, caractérisé en ce qu'il comprend en outre l'action d'incorporer de l'acide citrique dans la composition.

17. Procédé suivant l'une quelconque des revendications précédentes, caractérisé en ce que le composé actif biologiquement est de l'insuline, de l'hydrocortisone, de la prednisone, de l'interféron, un antibiotique, une enképhaline ou autre peptide, de la dopamine ou un agent antinéoplastique.